(19) Europäisches Patentamt
European Patent Office
Office européen des brevets

(11) Veröffentlichungsnummer: **0 346 593 A1**

# (12) EUROPÄISCHE PATENTANMELDUNG

(21) Anmeldenummer: **89106985.8**

(51) Int. Cl.4: **C07C 37/07 , C07C 39/15**

(22) Anmeldetag: **19.04.89**

(30) Priorität: **11.06.88 DE 3819963**

(43) Veröffentlichungstag der Anmeldung: **20.12.89 Patentblatt 89/51**

(84) Benannte Vertragsstaaten: **BE DE FR GB IT NL**

(71) Anmelder: **HÜLS AKTIENGESELLSCHAFT**
**Patentabteilung / PB 15 - Postfach 13 20**
**D-4370 Marl 1(DE)**

(72) Erfinder: **Kowalczik, Udo, Dr.**
**Camillo-Sitte-Weg 4**
**D-4630 Bochum 1(DE)**
Erfinder: **Bartmann, Martin, Dr.**
**Burgstrasse 35**
**D-4350 Recklinghausen(DE)**
Erfinder: **Finke, Jürgen, Dr.**
**Am Alten Sportplatz 17 a**
**D-4370 Marl(DE)**

## (54) Verfahren zur Herstellung von 4,4'-Dihydroxybiphenyl.

(57) Verfahren zur Herstellung von 4,4′-Dihydroxybiphenyl, wobei man 3,3′,5,5′-Tetra-tert.-butydiphenochinon mit 2,6-Di-tert.butylphenol in der Schmelze in Gegenwart eines 4-Dialkylaminopyridins reduziert und das entstehende Zwischenprodukt durch Zugabe einer phosphorhaltigen Säure oder ihres Derivates dealkyliert.

4,4′-Dihydroxybiphenyl ist ein Ausgangsprodukt zur Herstellung von flüssigkristallinen Polyestern.

EP 0 346 593 A1

## Verfahren zur Herstellung von 4,4′-Dihydroxybiphenyl

Gegenstand der Erfindung ist ein Verfahren zur Herstellung von 4,4′-Dihydroxybiphenyl, das beispielsweise zur Herstellung von flüssigkristallinen Polyestern benötigt wird.

Verfahren zur Herstellung von 4,4′-Dihydroxybiphenyl sind grundsätzlich bekannt. Ein Weg besteht darin, Biphenyl zu sulfonieren und anschließend zu verseifen. Dieses Verfahren ist nicht nur aufwendig sondern bringt auch erhebliche Entsorgungsprobleme mit sich. Daher bevorzugt man die Herstellung aus 3,3′,5,5′-Tetra-tert.-butyldiphenochinon. Beispielsweise beschreibt die US-PS 3 631 208 ein Verfahren zur Herstellung von 4,4′-Dihydroxybiphenyl durch Reduktion des Tetra-tert.-butyldiphenochinons mit 2,6-Di-tert.-butylphenol in Gegenwart eines Amins und anschließende Dealkylierung in Gegenwart eines Aluminumphenolates bei Temperaturen von etwa 280 °C. Das Zwischenprodukt 3,3′,5,5′-Tetra-tert.-butyl-4,4′-dihydroxybiphenyl wird mit einem geeigneten Antilösungsmittel ausgefällt und gegebenenfalls umkristallisiert. Eine Variante wird in der SU-PS 10 11 626 beschrieben; dort wird die Dealkylierung in Dekalin, Undecan oder Tridecan in Gegenwart eines Trialkylphenolats durchgeführt.

Die US-PS 4 482 755 lehrt die Reduktion des 3,3′,5,5′-Tetra-tert.-butyldiphenochinons mit Hilfe eines heterogenen Katalysators (z. B. Pd/C). Nach seiner Abtrennung erfolgt die Dealkylierung mit Hilfe eines sauren Ionenaustauschers. Nachteil dieser Verfahrensweise ist die Bildung von erheblichen Mengen an Nebenprodukten. Die sauer katalysierte Dealkylierung kann auch mit Schwefelsäure (JP-OS 83/189 127 bzw. US-PS 4 354 047) oder p-Toluolsulfonsäure in einem geeigneten Lösungsmittel (JP-OS 85/23 338 bzw. US-PS 4 354 048) durchgeführt werden.

Nach dem Stand der Technik ist es also erforderlich, die Reaktion in einem Lösungsmittel durchzuführen und das intermediär gebildete Zwischenprodukt 3,3′,5,5′-Tetra-tert.-butyl-4,4′-dihydroxybiphenyl zu isolieren. Beide Maßnahmen sind aufwendig und erschweren den Zugang zum Zielprodukt.

Aufgabe der vorliegenden Erfindung war es, ein kostengünstiges, einfacheres Verfahren zur Herstellung von 4,4′-Dihydroxybiphenyl aus 3,3′,5,5′-Tetra-tert.-butyldiphenochinon (DPCH) zu entwickeln.

Dies ist jetzt überraschend gelungen. Es konnte gezeigt werden, daß die Reduktion des Diphenochinons mit 2,6-Di-tert.-butylphenol (2 -10 Mol/Mol DPCH) durch Zugabe katalytischer Mengen eines 4-Dialkylaminopyridins (0,003 - 0,02 Mol/Mol DPCH), vorzugsweise bei Temperaturen von 240 - 280 °C, erleichtert wird. Die anschließende Zugabe einer - bezogen auf das 4-Dialkylaminopyridin äquimolaren - Menge einer phosphorhaltigen Säure oder deren Derivat und Erhitzen auf 280 -320 °C führt auf einfache Weise zum gewünschten 4,4′-Dihydroxybiphenyl. Auf ein Lösungsmittel und auf die Isolierung des Zwischenproduktes kann verzichtet werden.

Gegenüber dem Stand der Technik ergeben sich somit folgende Vorteile:

- Lösungsmittelfreies Verfahren, sowohl bei der Reduktion als auch bei der Dealkylierung
- Aufwendige Recyclingstufen zur Rückgewinnung der bisher benötigten Hilfsstoffe (z. B. Pyridin) können entfallen
- Effiziente Katalyse der Umsetzung des Diphenochinons mit 2,6-Di-tert.-butylphenol
- Keine Isolierung des Zwischenproduktes erforderlich
- Das Diphenochinon und 2,6-Di-tert.-butylphenol bilden zusammen das Zwischenprodukt; somit keine Bildung von Nebenprodukten und hoher Reinheitsgrad des erhaltenen Endproduktes

Das erfindungsgemäße Verfahren besteht darin, daß man zunächst ein Gemisch aus 3,3′,5,5′-Tetra-tert.butyldiphenochinon, 2,6-Di-tert.-butylphenol und dem 4-Dialkylaminopyridin auf 240 bis 280 °C erhitzt. Selbstverständlich ist es auch möglich, das 4-Dialkylaminopyridin erst bei höheren Temperaturen zuzusetzen. Hierdurch wird aber kein Vorteil erzielt. Die Entfärbung der Reaktionsmischung zeigt an, daß die Reduktion abgeschlossen ist. Nunmehr kommt es darauf an, das gegebenenfalls im Überschuß eingesetzte 2,5-Di-tert-butylphenol möglichst quantitativ abzudestillieren. Hierzu erhöht man zweckmäßigerweise die Temperatur der Reaktionsmischung kurzzeitig auf etwa 280 °C.

Die anschließende Dealkylierung erfolgt zweckmäßigerweise, indem man dem Reaktionsgemisch bei einer Temperatur von 240 °C die phosphorhaltige Verbindung zusetzt und langsam die Temperatur steigert, bis die Gasentwicklung abgeschlossen ist. Üblicherweise ist dies in einem Temperaturbereich bis 320 °C der Fall.

Die Isolierung des Produktes erfolgt beispielsweise durch Sublimation oder Umkristallisation.

Geeignete 4-Dialkylaminopyridine weisen die Formel

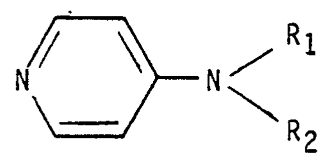

auf. Hierbei sind $R_1$ und $R_2$ unabhängig voneinander Alkylreste mit 1 bis 6 C-Atomen oder bilden zusammen mit dem benachbarten Stickstoffatom einen Pyrrolidin- oder Piperidinring. Vorzugsweise setzt man 4-Dimethylaminopyridin, 4-Di-n-butylaminopyridin, 4-Di-n-hexylaminopyridin oder 4-Piperidinylpyridin ein.

Als Katalysator für die Dealkylierung eignen sich phosphorhaltige Säuren und deren Derivate wie Phosphorsäureester und Phosphorsäuresalze. Bevorzugt sind Phosphorsäuren der allgemeinen Formel $H_3PO_n$ mit n = 2, 3 oder 4.

Beispiel 1

28,6 g (70 mmol) 3,3′,5,5′-Tetra-tert.-butyldiphenochinon, 29 g (140 mmol) 2,6-Di-tert.-butylphenol und 0,25 g (1,2 mmol) 4-Dibutylaminopyridin werden unter Stickstoff auf 255 °C erhitzt. Die nach 30 Minuten entfärbte Reaktionslösung wird dann mit 0,16 g (1,2 mmol) hypophosphoriger Säure (50%ig) versetzt und die Temperatur langsam auf 300 °C erhöht. Nach Beendigung der Gasentwicklung wird das Produkt durch Sublimation bei 1 mbar und 300 °C gewonnen.
Ausbeute: 24,8 g (> 95 % d. Th.)
Smp.: 278 - 280 °C

Beispiel 2

28,6 g (70 mmol) 3,3′,5,5′-Tetra-tert.-butyldiphenochinon, 57,8 g (280 mmol) 2,6-Di-tert.-butylphenol und 0,04 g (0,33 mmol) 4-Dimethylaminopyridin werden unter Stickstoff auf 260 °C erhitzt. Die nach 30 Minuten entfärbte Reaktionsmischung wird auf 280 °C erhitzt, das überschüssige 2,6-Di-tert.-butylphenol abdestilliert, und die Schmelze anschließend bei 250 °C mit 0,03 g (0,33 mmol) phosphoriger Säure versetzt. Erwärmen auf 280 °C führt zur Isobuten-Abspaltung und Bildung des leicht beigen Produktes, das aus Ethanol umkristallisiert wird.
Ausbeute: 33,9 g (> 90 %)
Smp.: 278 - 280 °C

**Ansprüche**

1. Verfahren zur Herstellung von 4,4′-Dihydroxybiphenyl durch Reduktion des 3,3′,5,5′-Tetra-tert.-butyldiphenochinons mit 2,6-Di-tert.-butylphenol und anschließende Dealkylierung des zwischenzeitlich gebildeten 3,3′,5,5′-Tetra-tert.butyl-4,4′-dihydroxybiphenyls bei erhöhter Temperatur,
dadurch gekennzeichnet,
daß man
a) die Reaktion in der Schmelze durchführt,
b) zur Reduktion ein 4-Dialkylaminopyridin der Formel

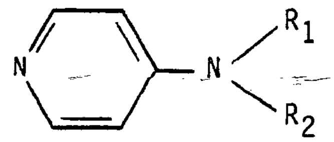

zusetzt, wobei $R_1$ und $R_2$ unabhängig voneinander eine Alkylgruppe mit 1 bis 6 C-Atomen bedeuten oder $R_1$ und $R_2$ zusammen mit dem benachbarten Stickstoffatom einen Pyrrolidin- oder Piperidinrest bilden, und
c) die Dealkylierung in Gegenwart einer phosphorhaltigen Säure oder einem ihrer Derivate durchführt.

2. Verfahren nach Anspruch 1,
dadurch gekennzeichnet,
daß man zur Reduktion
4-Dimethylaminopyridin,
4-Di-n-butylaminopyridin,
4-Di-n-hexylaminopyridin oder
4-Piperidinylpyridin
zusetzt.

3. Verfahren nach Anspruch 1,
dadurch gekennzeichnet,
daß zur Dealkylierung eine vom Phosphor abgeleitete Säure der allgemeinen Formel $H_3PO_n$ mit n = 2, 3 oder 4 verwendet wird.

4. Verfahren nach den Ansprüchen 1 bis 3,
dadurch gekennzeichnet,
daß man pro Mol 3,3′,5,5′-Tetra-tert.-butyldiphenochinon
a) 2 - 10 Mol 2,6-Di-tert.-butylphenol,
b) 0,003 - 0,02 Mol Dialkylaminopyridin und
c) 0,003 - 0,02 Mol phosphorhaltige Säure
einsetzt.

5. Verfahren nach den Ansprüchen 1 bis 4,
dadurch gekennzeichnet,
daß man
a) die Reduktion in einem Temperaturbereich von 240 - 280 °C und
b) die Dealkylierung in einem Bereich von 280 - 320 °C
durchführt.

Europäisches Patentamt

# EUROPÄISCHER RECHERCHENBERICHT

Nummer der Anmeldung

EP 89 10 6985

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CH-A- 655 088 (KADYROVA et al.)<br>* Anspruch *<br>----- | 1 | C 07 C 37/07<br>C 07 C 39/15 |
| | | | **RECHERCHIERTE SACHGEBIETE (Int. Cl.4)** |
| | | | C 07 C 37/00<br>C 07 C 39/00 |

Der vorliegende Recherchenbericht wurde für alle Patentansprüche erstellt

| Recherchenort | Abschlußdatum der Recherche | Prüfer |
|---|---|---|
| DEN HAAG | 13-09-1989 | KLAG M.J. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)